# EUROPÄISCHE PATENTANMELDUNG

(11) **EP 1 482 286 A1**
(43) Veröffentlichungstag der Anmeldung: **01.12.2004**
(21) Anmeldenummer: 03405376.9
(22) Anmeldetag: 26.05.2003
(51) Int. Cl.: G01G 19/38, G01N 1/04

(54) **Vorrichtung zum automatischen Transferieren und Dosieren eines schüttfähigen, insbesondere pulverförmigen Materials**

(71) Anmelder: METROHM AG, 9100 Herisau (CH)
(72) Erfinder: Kirschenbühler, Peter, 9100 Herisau (CH)
(74) Vertreter: Müller, Christoph Emanuel

(57) **Zusammenfassung**

Die Erfindung betrifft ein Behältnis 1 für schüttfähige, insbesondere pulverförmige Materialien M, zur Verwendung an einem Roboterarm R, mit einer Fördervorrichtung 3 in einem zylindrischen Gehäuse 2, wobei das zylindrische Gehäuse 2 als Aufnahmeraum für das Material M ausgebildet is, in welchem sich die Fördervorrichtung 3 zum Aufnehmen und Abgeben des Materials M in beide Richtungen quer zur Längsachse der Fördervorrichtung antreibbar angeordnet ist.

## Beschreibung

Die Erfindung betrifft Behältnisse zum Transferieren und Dosieren von schüttfähigen, insbesondere pulverförmige Materialien gemäss dem Oberbegriff von Anspruch 1, Vorrichtungen zum automatischen Transferieren und Dosieren von schüttfähigen, insbesondere pulverförmigen Materialien gemäss dem Oberbegriff von Anspruch 8, Kupplungsvorrichtungen gemäss dem Oberbegriff von Anspruch 11 sowie Verfahren zum Transferieren und Dosieren gemäss dem Oberbegriff von Anspruch 12.

Automatische Dosiervorrichtungen für pulverförmige Materialien sind insbesondere im Bereich der Chemietechnik an sich bekannt.

Die EP-A2-0 287 708 offenbart eine computergesteuerte Vorrichtung zum Abwiegen von Farbstoffen. Hierbei wird eine Mehrzahl von Silos verwendet, welche jeweils an ihrem unteren Ende über eine Förderschnecke verfügen. Ein beweglicher Schlitten mit einer Antriebseinrichtung kann die einzelnen Silos anfahren und die Antriebseinrichtung mit der jeweiligen Förderschnecke verbunden werden. Auf der gegenüberliegenden Seite der Silos befindet sich - ebenfalls auf dem Schlitten - eine Waage, in welche das Farbstoffpulver hineingefördert wird. Nachteilig bei dieser Vorrichtung ist insbesondere die Notwendigkeit einer Vielzahl fest installierter, separater Förderschnecken, wodurch sich eine eingeschränkte Flexibilität der Vorrichtung ergibt. Auch ist der hohe konstruktive Gesamtaufwand der Vorrichtung durch den komplexen Aufbau der Silos nachteilig.

Die EP-A1-0 446 398 offenbart ebenfalls eine computergesteuerte Vorrichtung zum Dosieren von pulverförmigen Materialien. Auch bei diesem System ist eine Vielzahl von Silos vorgesehen, welche jeweils in ihrem unteren Bereich über eine Förderschnecke verfügen. Gemäss der Vorgabe einer Steuereinheit fährt ein Wägeschlitten die einzelnen Silos an und empfängt die vorbestimmte Menge des jeweiligen Materials. Zusätzlich ist ein Roboterarm vorgesehen, welcher ein Wägegefäss auf den Wägeschlitten aufsetzen und wieder von diesem abnehmen kann.
Eine solche Anordnung eignet sich nicht zum möglichst variablen Transferieren von pulverförmigen Materialien.

Es ist daher eine Aufgabe der Erfindung, die Nachteile des Bekannten zu vermeiden; insbesondere also eine Vorrichtung zum automatischen Dosieren von schüttfähigen, insbesondere pulverförmigen Materialien zur Verfügung zu stellen, welche sich durch allgemein hohe Variabilität auszeichnet. Es ist insbesondere eine weitere Aufgabe der Erfindung, das Fördern und Dosieren verschiedenster schüttfähiger, insbesondere pulverförmiger Materialien bei möglichst geringem konstruktivem Aufwand insbesondere der Vorratsbehältnisse zu ermöglichen.

Erfindungsgemäss werden diese Aufgaben mit einem Behältnis und einer Vorrichtung zum insbesondere automatischen Transferieren und Dosieren von schüttfähigen, insbesondere pulverförmigen Materialien, einer Kupplungsvorrichtung und einem Verfahren gemäss den Kennzeichen der unabhängigen Ansprüche gelöst.

Die Erfindung betrifft ein Behältnis zum Transferieren und Dosieren eines schüttfähigen, insbesondere pulverförmigen Materials, mit einer Fördervorrichtung in einem zylindrischen Gehäuse, wobei das zylindrische Gehäuse als Aufnahmeraum für das Material ausgebildet ist, in welchem die Fördervorrichtung zum Aufnehmen und Abgeben des Materials in beide Richtungen quer zur Längsachse der Fördervorrichtung antreibbar angeordnet ist.
Der Aussendurchmesser der Fördervorrichtung ist insbesondere derart auf den Innendurchmesser des zylindrischen Gehäuses abgestimmt, dass die Fördervorrichtung in dem zylindrischen Gehäuse drehbar ist, ein unkontrolliertes seitliches Entweichen des zu fördernden Materials jedoch durch einen passgenauen Sitz der Fördervorrichtung in dem zylindrischen Gehäuse vermieden wird. Besonders vorteilhaft ist das Behältnis insbesondere zu Reinigungszwecken zerlegbar und wiederverwertbar. Vorteilhafterweise besteht das Behältnis aus einem chemisch inerten und leicht spritzbaren Kunststoff oder Mischungen derartiger Kunststoffe, insbesondere Polyvinylchlorid (PVC), Polyethylen (PE), Polyurethan (PU), Polyamid (Polyamid).
Als Fördervorrichtung wird besonders bevorzugt eine Förderschraube eingesetzt. Je nach zu förderndem Material kann selbstverständlich bei Bedarf eine Fördervorrichtung, insbesondere eine Förderschraube gewählt werden, welche den speziellen Eigenschaften des zu fördernden Materials gerecht wird. Insbesondere kann der Steigungswinkel der Ebenen einer Förderschraube sowie die Rauhigkeit der Oberflächen an das jeweils zu fördernde Material angepasst sein, um ein optimales Schüttverhalten zu gewährleisten. Zudem kann bei Bedarf der Roboterarm mit einer Anfahr- und/oder Abbremsrampe bewegt werden. Alternativ oder zusätzlich kann nach der Aufnahme des Materials in einem aus dem Vorratsbehältnis ausgefahrenen Zustand die Fördervorrichtung noch kurz in die Aufnahmerichtung gedreht werden.
Im Betrieb wird das zu fördernde Material in das Behältnis durch Drehung der Fördervorrichtung in eine der beiden möglichen Richtungen quer zur Längsachse der Fördervorrichtung aufgenommen. Die Abgabe des zu fördernden Materials aus dem Behältnis ist durch Drehung der Fördervorrichtung in die entgegengesetzte Richtung möglich.

In einer bevorzugten Ausführungsform ist in einem Endbereich des Behältnisses eine Kupplungsvorrichtung zur Aufnahme an einem Roboterarm vorgesehen, wobei die Kupplungsvorrichtung insbesondere das zylindrische Gehäuse des Behältnisses verdrehgesichert fixiert. Durch die Kombination eines erfindungsgemässen Behältnisses mit einem Roboterarm ist eine besonders hohe Variabilität und Vielseitigkeit der Anwendung erzielbar.

In einer weiteren besonders bevorzugten Ausführungsform verfügt die Fördervorrichtung darüber hinaus in ihrem der Kupplungsvorrichtung zur Aufnahme an einem Roboterarm zugewandten Endbereich über eine Kupplungsvorrichtung zur Verbindung mit einer Antriebsvorrichtung. Diese Antriebsvorrichtung kann hierbei direkt im Roboteram vorgesehen sein. Es ist aber beispielsweise auch eine Antriebswelle zur Übertragung geeignet, so dass die Antriebseinheit selbst weiter entfernt, beispielsweise in einer Basisstation des Roboters untergebracht sein kann. An dem Roboterarm kann optional auch eine Aufnahme vorgesehen sein, um Probengefässe und/oder Vorratsbehältnisse, insbesondere Rollrandgefässe zu fixieren und zu transportieren.
Besonders vorteilhaft wird das erfindungsgemässe Behältnis an einem Roboterarm im Zusammenspiel mit einer automatischen Probenverarbeitungsanlage ("sample processor") an sich bekannter Bauart, insbesondere Mikroprozessor-gesteuert eingesetzt. Im Betrieb befindet sich dann ein zu dosierendes Material in einem Gefäss in einer Halterung der automatischen Probenverarbeitungsanlage. Selbstverständlich können auch mehrere Gefässe mit unterschiedlichsten zu dosierenden Materialien auf der Probenverarbeitungsanlage angeordnet sein. Ebenfalls in einer Halterung der automatischen Probenverarbeitungsanlage befindet sich ein Vorrat an Behältnissen zum Transferieren und Dosieren des Materials. An einer externen Position oder ebenfalls in der automatischen Probenverarbeitungsanlage befindet sich eine Wägevorrichtung, auf welcher insbesondere auch durch den Roboterarm ein Wägegefäss positioniert werden kann. Der Roboterarm nimmt aus der Halterung mit dem Vorrat an Behältnissen ein Behältnis auf und fährt anschliessend die Position der automatischen Probenverarbeitungsanlage an, an der sich das zu dosierende Material befindet. Der Roboterarm mit dem Behältnis wird in das zu dosierende Material abgesenkt und durch eine Drehung der Fördervorrichtung wird das Material in das Behältnis aufgenommen. Mit dem in das Behältnis aufgenommen Material fährt der Roboterarm anschliessend die Position der Wägevorrichtung an. Durch Drehen, insbesondere langsames Drehen der Fördervorrichtung in die entgegengesetzte Richtung wird das zu dosierende Material auf die Wägevorrichtung aus dem Behältnis abgegeben. Besonders vorteilhaft verfügt die Anordnung über Kommunikationsmittel zwischen Wägevorrichtung und automatischer Probenverarbeitungsanlage. Hierduch kann laufend das Gewicht der bereits abgegebenen Menge des Materials an die automatische Probenverarbeitungsanlage übermittelt werden und die Drehung der Fördervorrichtung bei Erreichen des voreingstellten, zu erreichenden Gewichts beendet werden. Optional verfügt die automatische Probenverarbeitungsanlage darüber hinaus über eine elektronische Steuerung, welche es ermöglicht, die Drehung der Fördervorrichtung in Abhängigkeit von dem von der Waage detektierten Gewicht insbesondere stufenlos zu steuern. Es ist besonders vorteilhaft, die Dosierinkremente kleiner zu wählen und/oder eine langsamere Drehung der Fördervorrichtung zu wählen, je mehr die bereits abgegebene Menge des Materials sich dem vorbestimmten Gewicht nähert. Hierdurch wird ein möglichst exaktes Erreichen des vorbestimmten Gewichts gewährleistet. Optional wird nach dem Abwiegevorgang das Wägegefäss beispielsweise mit einer Aufnahmegabel des Roboterarms von der Wägevorrichtung genommen und anschliessend ein leeres Wägegefäss auf der Wägevorrichtung positioniert. Durch die beschriebene Anordnung ist ein vollständig automatisierter Betrieb auch beim Dosieren von mehreren Proben möglich.

Gemäss einer weiteren bevorzugten Ausführungsform weist das zylindrische Gehäuse des Behältnisses eine Profilierung, insbesondere einen Vorsprung oder eine Aussparung auf, welche das automatisierte Abstreifen des Behältnisses von einem Roboterarm ermöglicht. Beim Dosieren mehrerer unterschiedlicher Materialien ist ein Wechseln der Behältnisse notwendig, um Kreuz-Kontaminationen zu vermeiden. Bevorzugterweise erfolgt ein solcher Wechsel der Behältnisse automatisch. Als Profilierung ist beispielsweise einen erhabener Ring auf dem zylindrischen Gehäuse geeignet. Das anschliessende Aufnehmen eines neuen Behältnisses erfolgt wie vorstehend beschrieben.

Gemäss einer weiteren bevorzugten Ausführungsform ist die Kupplungsvorrichtung zur Verbindung der Fördervorrichtung mit einer Antriebseinheit aus mechanisch ineinandergreifenden Teilen ausgebildet. Hierbei sind besonders vorteilhaft Schlitzschrauben-, Kreuzschlitzschrauben- oder Torque-Verbindungen verwendbar.

Gemäss einer weiteren, besonders bevorzugten Ausführungsform ist die Kupplungsvorrichtung zur Aufnahme des Behältnisses an einem Roboterarm konusförmig ausgebildet. Zum Konus des Behältnisses kompatibel ist ein Konus des Roboterarms. Bevorzugterweise ist der Konus des Behältnisses als konusförmige Vertiefung und der Konus des Roboterarms als erhabener Konus ausgebildet. Im Betriebszustand sitzt das Behältnis presspassgenau auf dem Roboterarm. Eine derartige Kupplungsvorrichtung bietet durch ihre mechanische Einfachheit einen guten Kompromiss aus Stabilität der Verbindung und leichter automatischer Abstreifbarkeit.

Gemäss einem besonders bevorzugten Ausführungsbeispiel weist das Behältnis im Bereich der Kupplungsvorrichtung eine konusförmige Vertiefung mit einem Öffnungswinkel zwischen 0,5° und 15°, vorzugsweise zwischen 2° und 5°, besonders bevorzugt von ca. 2,8° auf, welche zu einem Konus des Roboterarms kompatibel ist. Ein Öffnungswinkel zwischen 0,5° und 15°, vorzugsweise zwischen 2° und 5°, besonders bevorzugt von ca. 2,8° hat sich als besonders vorteilhafter Kompromiss aus Stärke der Verbindung zwischen Behältnis und Roboterarm einerseits und der notwendigen Abstreifbarkeit zum Austausch des Behältnisses andererseits erwiesen. Eine individuelle Anpassung des Öffnungswinkels ist selbstverständlich insbesondere je nach Material des Behältnisses und des Konus des Roboterarms möglich.

Die erfindungsgemässen Behältnisse sind besonders vorteilhaft in Vorrichtungen zum automatischen Transferieren und Dosieren von schüttfähigen, insbesondere pulverförmigen Materialien einsetzbar. Derartige Vorrichtungen besitzen vorteilhafterweise mindestens einen Roboterarm zur Aufnahme eines erfindungsgemässen Behältnisses. Durch die automatisierbare und leichte Austauschbarkeit des Behältnisses wird eine hohe Vielseitigkeit der Vorrichtung erzielt; insbesondere ist die Vermeidung von Kreuz-Kontaminationen durch den Einsatz von Behältnissen je zu dosierendem Material leicht zu gewährleisten.

In einem besonders bevorzugten Ausführungsbeispiel verfügt die Vorrichtung zum automatischen Transferieren und Dosieren von schüttfähigen, insbesondere pulverförmigen Materialien über eine elektronische Rückkoppelschleife zwischen einer automatischen Probenverarbeitungsanlage und einer Wägevorrichtung. Hierdurch wird eine besonders exakte Dosierung eines voreingestellten Gewichts ermöglicht. Die hierfür notwendigen Kommunikationsmittel zwischen der Wägevorrichtung und der automatischen Probenverarbeitungsanlage können insbesondere auch drahtlos ausgebildet sein. Durch eine elektronische Rückkoppelschleife kann laufend das Gewicht der bereits abgegebenen Menge des Materials an die automatische Probenverarbeitungsanlage übermittelt werden und die Drehung der Fördervorrichtung bei Erreichen des voreingstellten, zu erreichenden Gewichts beendet werden. Optional verfügt die automatische Probenverarbeitungsanlage darüber hinaus über eine elektronische Steuerung, welche es ermöglicht, die Geschwindkeit der Drehung der Fördervorrichtung und/oder das Dosierinkrement in Abhängigkeit von dem von der Waage detektierten Gewicht insbesondere stufenlos zu steuern. Hierbei ist es besonders vorteilhaft, die Dosierinkremente durch eine langsamere Drehung der Fördervorrichtung kleiner zu wählen, je mehr die bereits abgegebene Menge des Materials sich dem vorbestimmten Gewicht nähert. Hierdurch wird ein möglichst exaktes Erreichen des vorbestimmten Gewichts gewährleistet.

In einem weiteren besonders bevorzugten Ausführungsbeispiel ist die Wägevorrichtung in dem Roboterarm, insbesondere direkt benachbart zu dem Behältnis angeordnet. Hierdurch können alle Transporte des Wägegefässes auf eine externe Wägeposition und von dieser zurück entfallen. Zudem ist ein besonders sensibles Ansprechverhalten der Wägevorrichtung auf Gewichtsveränderungen in dem Behältnis und demzufolge eine besonders exakte Erreichbarkeit des voreingestellten Gewichts des zu dosierenden Materials erzielbar.

Die Verwendung eines Schneckenförderers als Transferbehältnis führt ersichtlicherweise zu einer flexiblen, auf verschiedene Weise einsetzbaren Transfer- und Dosieranordnung.

Im folgenden wird die Erfindung anhand von Zeichnungen und Ausführungsbeispielen näher erläutert, ohne dass die Erfindung auf diese zu beschränken ist. Hierbei zeigen:
- Fig. 1(a):: Behältnis und Roboterarm, separiert;
- Fig. 1(b):: Behältnis an Roboterarm, Aufnahme des zu dosierenden Materials;
- Fig. 1(c):: Behältnis an Roboterarm, Abgabe des zu dosierenden Materials;
- Fig. 1(d):: Behältnis, zerlegt
- Fig. 2:: Behältnis und Roboterarm mit integrierter Wägevorrichtung.

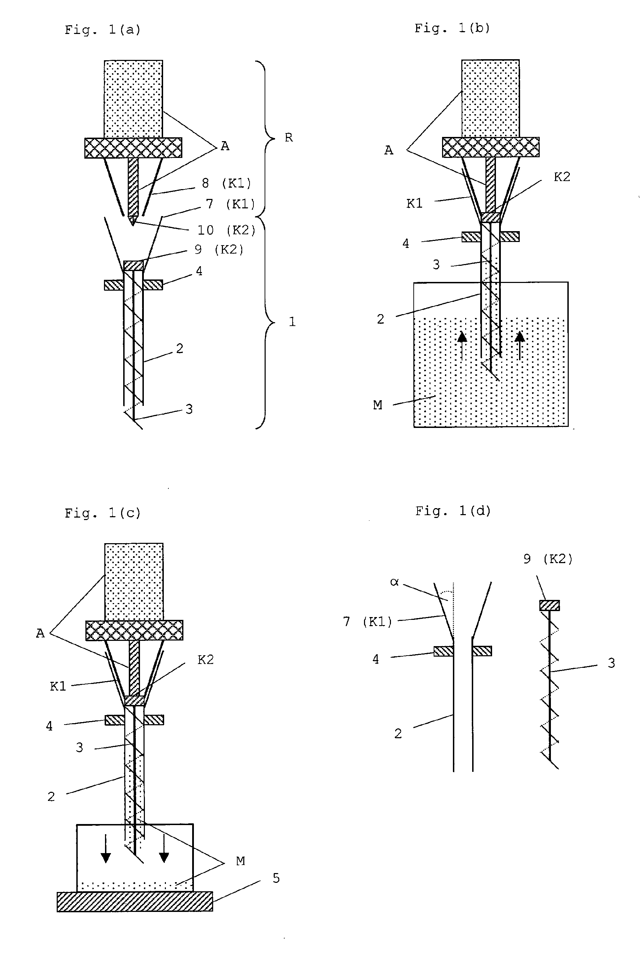

Fig. 1 zeigt ein Behältnis 1 zum Transferieren und Dosieren eines schüttfähigen, insbesondere pulverförmigen Materials sowie einen Roboterarm R zur Aufnahme des Behältnisses 1. Das Behältnis 1 verfügt über ein zylindrisches Gehäuse 2, in welchem eine Fördervorrichtung 3 in beide Richtungen quer zur Längsachse drehbar gelagert ist. Vorzugsweise werden als Fördervorrichtung 3 beispielsweise Förderschnecken bzw. Förderschrauben eingesetzt. Je nach zu förderndem Material kann selbstverständlich bei Bedarf eine Fördervorrichtung 3 gewählt werden, welche den speziellen Eigenschaften des zu fördernden Materials gerecht wird. Insbesondere kann der Steigungswinkel der Ebenen einer Förderschraube sowie die Rauhigkeit der Oberflächen an das jeweils zu fördernde Material angepasst sein, um ein optimales Schüttverhalten zu gewährleisten. Zudem kann bei Bedarf der Roboterarm R mit einer Anfahr- und/oder Abbremsrampe bewegt werden. Alternativ oder zusätzlich kann nach der Aufnahme des Materials in einem aus dem Vorratsbehältnis ausgefahrenen Zustand die Fördervorrichtung 3 noch kurz in die Aufnahmerichtung gedreht werden. Das Behältnis 1 besitzt optional eine Profilierung 4, beispielsweise einen das zylindrische Gehäuse 2 umlaufenden Ring. Diese Profilierung 4 ermöglicht ein automatisiertes Abstreifen des Behältnisses 1 von dem Roboterarm R. Es ist darüber hinaus eine Kupplungsvorrichtung K1 zur Aufnahme des Behältnisses 1 am Roboterarm R vorgesehen. In der gezeigten Ausführungsform ist die Kupplungsvorrichtung K1 am Roboterarm als Konus 8 und an dem Behältnis 1 als konusförmige Vertiefung 7 ausgebildet. Das Behältnis 1 passt in diesem Fall presspassgenau auf den Roboterarm R, wodurch das zylindrische Gehäuse 2 verdrehgesichert am Roboterarm R fixiert ist. Selbstverständlich sind auch andere Kupplungsvorrichtungen K1 wie beispielsweise Verschraubungen möglich. Zur Übertragung einer Drehbewegung von einem im Roboterarm R untergebrachten Antrieb A auf die Fördervorrichtung 3 ist eine weitere Kupplungsvorrichtungen K2 vorgesehen. Auf Seiten des Behältnisses 1 ist die Kupplungsvorrichtung K2 beispielsweise als Schlitz 9, auf Seiten des Roboterarms R als zu diesem Schlitz kompatibler Vorsprung 10 ausgebildet. Als mechanisch besonders vorteilhafte Vergundungen sind besonders vorteilhaft Schlitz-, Kreuzschlitz- und Torque-Verbindungen geeignet.

Fig. 1(b) zeigt ein am Roboterarm R befindliches Behältnis 1 während der Aufnahme eines zu fördernden bzw. dosierenden Materials M. Die Fixierung des Behältnisses 1 am Roboterarm ist über die Kupplungsvorrichtung K1 in bereits beschriebener Art und Weise gewährleistet. Die Kupplungsvorrichtung K2 stellt die Übertragung einer Drehbewegung des Antriebs A auf die Fördervorrichtung 3 sicher. Nicht explizit gezeigt ist die Aufnahme eines Behältnisses 1 durch den Roboterarm. Dies kann in an sich bekannter Weise automatisiert erfolgen, beispielsweise an einer Position einer automatischen Probenverarbeitungsanlage ("sample processor"), wo sich ein Vorrat an Behältnissen befindet. Selbstverständlich kann sich dieser Vorrat auch an einer Position extern von der automatischen Probenverarbeitungsanlage befinden. Die Aufnahme des Behältnisses ist durch den presspassgenauen Sitz der Kupplungsvorrichtung K1 durch einfaches Aufpressen des Roboterarms auf das Behältnis 1 leicht möglich. Im Betrieb wird das zu fördernde Material M in das Behältnis 1 durch eine aufwärts gerichtete Drehung der Fördervorrichtung aufgenommen. Je nach Ausbildung des Behältnisses 1 und der Fördervorrichtung 3 können insbesondere Mengen von 10mg bis 5g aufgenommen werden; eine Anpassung der Kapazität ist durch konstruktive Routinearbeiten leicht möglich.

Fig. 1(c) zeigt das Abgeben des zu fördernden Materials M beispielsweise an einer anderen Position einer automatischen Probenverarbeitungsanlage oder einer externen Position. Durch eine abwärts gerichtete Drehbewegung der Fördervorrichtung 3 wird das zu fördernde Material M aus dem Behältnis 1 in ein auf einer Wägevorrichtung 5 angeordnetes Gefäss entlassen. Besonders bevorzugt sind Kommunikationsmittel zwischen automatischer Probenverarbeitungsanlage bzw. Antriebsvorrichtung A und der Wägevorrichtung 5 vorgesehen, so dass bei Erreichen des voreingestellten Gewichts die Drehbewegung der Fördervorrichtung 3 gestoppt wird. Bevorzugterweise ist hierfür eine elektronische Rückkoppelschleife vorgesehen, über welche insbesondere auch die Fördergeschwindigkeit vorzugsweise stufenlos geregelt werden kann, so dass bei Annäherung an das Sollgewicht die Fördergeschwindigkeit verlangsamt werden kann. Hierdurch kann ein besonders präzises Erreichen des Sollgewichts gewährleistet werden.

In Fig. 1(d) sind das zylindrische Gehäuse 2 und die Fördervorrichtung 3 eines Behältnisses 1 voneinander separiert gezeigt. Das Behältnis 1 ist bevorzugterweise zumindest in diese beiden Bestandteile reversibel zerlegbar, um insbesondere eine effiziente Reinigung und damit die Wiederverwertbarkeit des Behältnisses 1 zu gewährleisten. Die Kupplungsvorrichtung K1 ist bevorzugt als konusfömige Vertiefung ausgebildet, welche einen Öffnungswinkel (α) zwischen 0,5° und 15°, vorzugsweise zwischen 2° und 5°, besonders bevorzugt von ca. 2,8° aufweist.

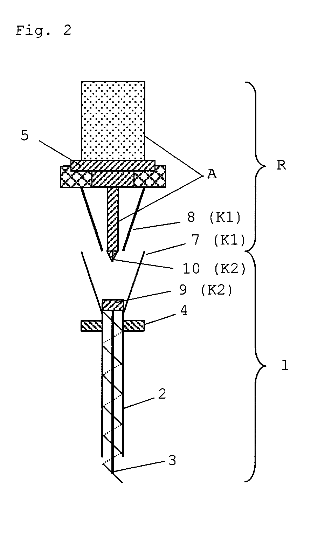

In Fig. 2 ist eine besonders bevorzugte Ausführungsform gezeigt, wobei die Wägevorrichtung 5 in den Roboterarm integriert ist. Hierdurch ist ein besonders sensibles Ansprechverhalten der Wägevorrichtung 5 auf Gewichtsveränderungen in dem Behältnis 1 und demzufolge eine besonders exakte Erreichbarkeit des voreingestellten Gewichts des zu dosierenden Materials M erreichbar.

## Patentansprüche

1. Behältnis (1) zum Transferieren und Dosieren eines schüttfähigen, insbesondere pulverförmigen Materials (M), mit einer Fördervorrichtung (3) in einem zylindrischen Gehäuse (2),
**dadurch gekennzeichnet, dass**
das zylindrische Gehäuse (2) als Aufnahmeraum für das Material (M) ausgebildet ist, in welchem die Fördervorrichtung (3) zum Aufnehmen und Abgeben des Materials (M) in beide Richtungen quer zur Längsachse der Fördervorrichtung (3) antreibbar angeordnet ist.

2. Behältnis (1) nach Anspruch 1,
**dadurch gekennzeichnet, dass** in einem Endbereich des Behältnisses (1) eine Kupplungsvorrichtung (K1) zur Aufnahme an einem Roboterarm (R) vorgesehen ist, wobei die Kupplungsvorrichtung (K1) das zylindrische Gehäuse (2) des Behältnisses (1) verdrehgesichert fixiert.

3. Behältnis (1) nach Anspruch 2,
**dadurch gekennzeichnet, dass** die Fördervorrichtung (3) in ihrem der Kupplungsvorrichtung (K1) zugewandten Endbereich über eine Kupplungsvorrichtung (K2) zur Verbindung mit einer Antriebsvorrichtung (A) verfügt.

4. Behältnis (1) nach einem der Ansprüche 2 oder 3,
**dadurch gekennzeichnet, dass** das zylindrische Gehäuse (2) eine Profilierung (4), insbesondere einen Vorsprung oder eine Aussparung aufweist, welche das automatisierte Abstreifen des Behältnisses (1) von einem Roboterarm (R) ermöglicht.

5. Behältnis (1) nach einem der Ansprüche 3 bis 4,
**dadurch gekennzeichnet, dass** die Kupplungsvorrichtung (K2) aus mechanisch ineinandergreifenden Teilen ausgebildet ist.

6. Behältnis (1) nach einem der Ansprüche 2 bis 5,
**dadurch gekennzeichnet, dass** die Kupplungsvorrichtung (K1) konusförmig ausgebildet ist.

7. Behältnis (1) nach einem der Ansprüche 2 bis 6,
**dadurch gekennzeichnet, dass** das Behältnis (1) im Bereich der Kupplungsvorrichtung (K1) eine konusförmige Vertiefung (7) mit einem Öffnungswinkel (α) zwischen 0,5° und 15°, vorzugsweise zwischen 2° und 5°, besonders bevorzugt von ca. 2,8° aufweist, welche zu einem Konus (8) des Roboterarms (R) kompatibel ist.

8. Vorrichtung (6) zum automatischen Transferieren und Dosieren von schüttfähigen, insbesondere pulverförmigen Materialien (M),
**dadurch gekennzeichnet, dass** ein Behältnis (1) gemäss einem der Ansprüche 1 bis 7 vorgesehen ist.

9. Vorrichtung (6) nach Anspruch 8,
**dadurch gekennzeichnet, dass** eine insbesondere elektronische Rückkoppelschleife zwischen einer automatischen Probenverarbeitungslage (S) und einer Wägevorrichtung (5) die Dosierung eines voreingestellten Gewichts ermöglicht.

10. Vorrichtung (6) nach Anspruch 9,
**dadurch gekennzeichnet, dass** die Wägevorrichtung (5) in dem Roboterarm (R) integriert ist.

11. Kupplungsvorrichtung (K1) zur Verbindung eines Behältnisses (1) mit einem Roboterarm (R),
**dadurch gekennzeichnet, dass** das Behältnis (1) eine konusförmige Vertiefung (7) mit einem Öffnungswinkel (α) zwischen 0,5° und 15°, vorzugsweise zwischen 2° und 5°, besonders bevorzugt von ca. 2,8° aufweist, welche zu einem Konus (8) des Roboterarms (R) kompatibel ist.

12. Verfahren zum Transferieren und Dosieren von schüttfähigen, insbesondere pulverförmigen Materialien (M), mit den folgenden Schritten:
(i) Bereitstellung eines Behältnisses (1) mit einer Fördervorrichtung (3) in einem zylindrischen Gehäuse (2), wobei das zylindrische Gehäuse (2) als Aufnahmeraum für das Material (M) ausgebildet ist, in welchem die Fördervorrichtung (3) zum Aufnehmen und Abgeben des Materials (M) in beide Richtungen quer zur Längsachse der Fördervorrichtung (3) antreibbar angeordnet ist;
(ii) Absenken des Behältnisses (1) vorzugsweise an einem Roboterarm (R) in das Material (M) und Aufnahme des Materials (M) durch Drehung der Fördervorrichtung (3) in eine erste Richtung quer zur Längsachse der Fördervorrichtung (3);
(iii) Transfer des Behältnisses (1) und Abgabe des Materials (M) auf eine Wägevorrichtung (5) und/oder kontrolliert durch eine insbesondere in einem Roboterarm angeordnete Wägevorrichtung (5), wobei die Abgabe des Materials (M) durch Drehung der Fördervorrichtung (3) in eine zweite Richtung quer zur Längsachse der Fördervorrichtung (3) erfolgt.
